(19) Europäisches Patentamt
European Patent Office
Office européen des brevets

(11) Veröffentlichungsnummer: **0 006 090**
**B1**

(12) **EUROPÄISCHE PATENTSCHRIFT**

(45) Veröffentlichungstag der Patentschrift: **01.09.82**

(21) Anmeldenummer: **78100148.2**

(22) Anmeldetag: **12.06.78**

(51) Int. Cl.³: **C 07 J 41/00,**
A 61 K 31/57,
C 07 J 43/00, A 61 K 31/58

# (54) Neue Kortikoide, Verfahren zu ihrer Herstellung und diese enthaltende pharmazeutische Präparate.

(43) Veröffentlichungstag der Anmeldung:
**09.01.80 Patentblatt 80/1**

(45) Bekanntmachung des Hinweises auf die Patenterteilung:
**01.09.82 Patentblatt 82/35**

(84) Benannte Vertragsstaaten:
**BE CH DE FR GB LU NL**

(56) Entgegenhaltungen:
**FR - A - 2 156 518**
**US - A - 3 436 388**

Die Akte enthält technische Angaben die nach dem Eingang der Anmeldung eingereicht wurden und die nicht in dieser Patentschrift enthalten sind.

(73) Patentinhaber: **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder: **Laurent, Henry, Dr.**
**Glambecker Weg 21**
**D-1000 Berlin 28 (DE)**
Erfinder: **Wiechert, Rudolf, Prof.**
**Petzower Strasse 8a**
**D-1000 Berlin 39 (DE)**
Erfinder: **Töpert, Michael, Dr.**
**Askaloner Weg 11**
**D-1000 Berlin 28 (DE)**
Erfinder: **Kapp, Joachim-Friedrich, Dr.**
**Ludolfinger Weg 51**
**D-1000 Berlin 28 (DE)**

**EP 0 006 090 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Neue Kortikoide, Verfahren zu ihrer Herstellung und diese enthaltende pharmazeutische Präparate

Die Erfindung betrifft neue Kortikoide, ein Verfahren zu ihrer Herstellung und pharmazeutische Präparate, die diese Kortikoide als Wirkstoff enthalten.

Die neuen Kortikoide sind gekennzeichnet durch die allgemeine Formel I

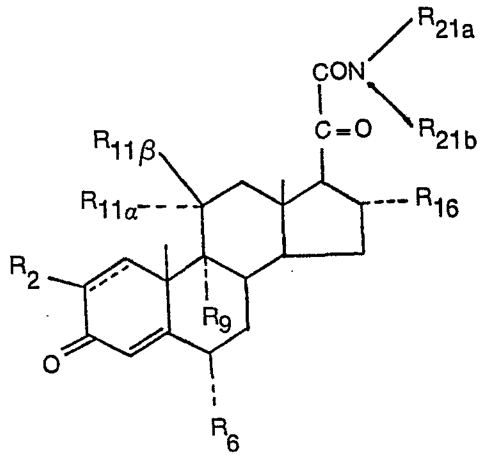

(I),

worin

die Bindung ...... eine Einfachbindung oder eine Doppelbindung

$R_2$ ein Wasserstoffatom oder ein Chloratom,

$R_6$ ein Wasserstoffatom oder ein Fluoratom und

$R_{16}$ ein Wasserstoffatom oder eine Methylgruppe bedeuten, worin

$R_9$ ein Wasserstoffatom, ein Fluoratom oder ein Chloratom und

$R_{11\alpha}$ und $R_{11\beta}$ gemeinsam ein Oxogruppe oder $R_{11\alpha}$ ein Wasserstoffatom und $R_{11\beta}$ eine Hydroxygruppe oder falls $R_9$ ein Chloratom ist auch ein Fluoratom oder ein Chloratom darstellen und worin

$R_{21a}$ und $R_{21b}$ gleich oder verschieden sind und Wasserstoff oder bis zu 8 Kohlenstoffatome enthaltende Alkylreste oder Cycloalkylreste oder gemeinsam einen gegebenenfalls durch Sauerstoff oder Stickstoff unterbrochenen bis zu 8 Kohlenstoffatome enthaltenden Alkylenrest darstellen.

Geeignete Reste $R_{21a}$ und/oder $R_{21b}$ sind zum Beispiel: Der Methylrest, der Äthylrest, der Propylrest, der Isopropylrest, der Butylrest, der sek.-Butylrest, der Pentylrest, der Cyclopentylrest, der Hexylrest oder der Cyclohexylrest.

Reste $R_{21a}$ und $R_{21b}$, die gemeinsam eine gegebenenfalls durch Sauerstoff unterbrochene Alkylengruppe bedeuten, sind zum Beispiel die Tetramethylengruppe, die Pentamethylengruppe, die 3-Oxopentamethylengruppe oder die 3-Azapentamethylengruppe.

Das erfindungsgemäße Verfahren zur Herstellung der neuen Kortikoide ist dadurch gekennzeichnet, daß man in an sich bekannter Weise

a) eine Carbonsäure der allgemeinen Formel II

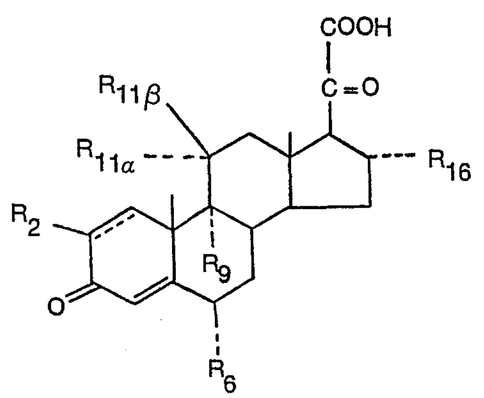

(II),

worin ....., $R_2$, $R_6$ $R_9$, $R_{11\alpha}$, $R_{11\beta}$ und $R_{16}$ die obengenannte Bedeutung besitzen, oder ein reaktionsfähiges Derivat dieser Säure mit eine Amin der allgemeinen Formel III

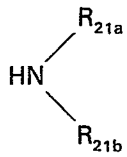

(III),

worin $R_{21a}$ und $R_{21b}$ die obengenannte Bedeutung besitzen, kondensiert, oder

b) zur Herstellung von Kortikoiden mit $R_9$ in der Bedeutung eines Chloratoms an die 9(11)-Doppelbindung einer Verbindung der allgemeinen Formel IV

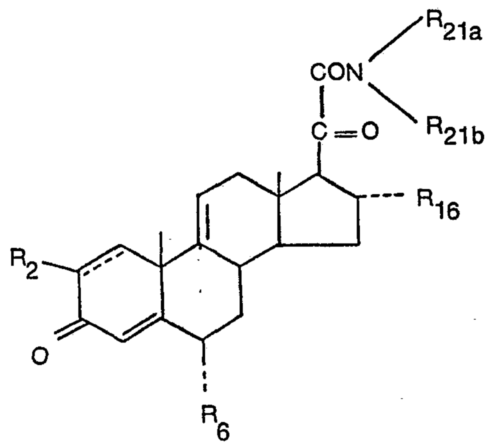

(IV),

worin $R_2$, $R_6$, $R_{16}$, $R_{21a}$ und $R_{21b}$ die obengenannte Bedeutung besitzen, unterchlorige Säure, Fluorchlorid oder Chlor anlagert.

c) Zur Herstellung von Kortikoiden mit $R_{11\beta}$ in der Bedeutung einer Hydroxygruppe und $R_9$ in der Bedeutung eines Fluoratoms oder eines Chloratoms den 9,11-Epoxydring einer Verbindung der allgemeinen Formel V

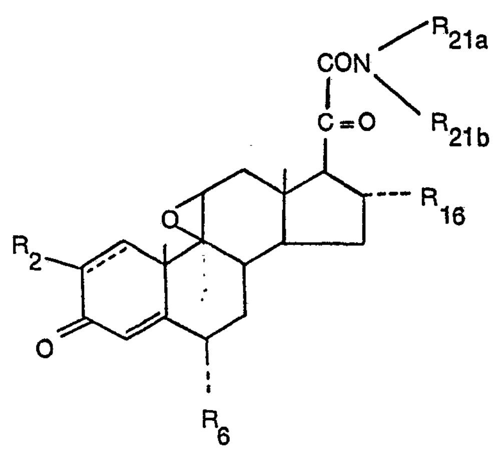

(V),

mit Fluorwasserstoff oder Chlorwasserstoff öffnet.

Das erfindungsgemäß Verfahren gemäß Verfahrensvariante a wird unter Bedingungen durchgeführt, die dem Fachmann wohl bekannt sind.

So kann man beispielsweise die Carbonsäuren der allgemeinen Formel II in Gegenwart von Katalysatoren, wie man sie bei der Amidherstellung üblicherweise verwendet (Dicyclohexylcarbodiimid, N,N-Carbonyldiimidazol, N-Äthoxycarbonyl-2-äthoxy-1,2-dihydrochinolin etc.) mit den Aminen der Formel III umsetzen.

Ferner ist es möglich, die Carbonsäuren, beispielsweise mittels Thionylchlorid, in die Säurechloride zu überführen und diese mit den Aminen umzusetzen.

Günstigere Ausbeuten erzielt man meist, wenn man die Carbonsäuren der allgemeinen Formel II — beispielsweise durch Umsetzen mit einen Chlorameisensäurealkylester — in ihre gemischten Anhydride überführt und auf diese die Amine einwirken läßt.

Ferner kann man Ester der Carbonsäuren der Formel II (so zum Beispiel Alkylester mit 1 bis 6 Kohlenstoffatomen in der Alkylgruppe) mit den Aminen der allgemeinen Formel III kondensieren.

Die Umsetzung kann in polaren oder unpolaren Lösungsmitteln durchgeführt werden. Geeignete

Lösungsmittel sind beispielsweise: Benzol, Toluol, Xylol, Tetrahydrofuran, Dioxan, Dimethoxyäthan, Chloroform, Dimethylformamid, Dimethylsulfoxyd oder Hexamethylphosphorsäuretriamid.

Die Reaktion wird vorzugsweise bei einer Reaktionstemperatur von —60° C bis +100° C durchgeführt.

Das erfindungsgemäße Verfahren gemäß Verfahrenvariante b und c erfolgt ebenfalls unter Bedingungen, die dem Fachmann wohl bekannt sind (siehe beispielsweise die US-Patentschrift 37 18 671).

Die neuen Kortikoide der allgemeinen Formel I besitzen eine gute antiphlogistische Wirksamkeit und sind in Gegensatz zu denjenigen des FR—A—2 156518 und des US—A 3,436,388 stark systemisch wirksam wie mit Hilfe des Rezeptorbindungstest (Lübke, K. et al., Angewandte Chemie (Engl. Ed.) *15* 741—748, 1976) nachgewiesen werden kann.

Die neuen Kortikoide sind in Kombination mit den in der galenischen Pharmazie üblichen Trägermitteln gut geeignet zur Behandlung zum Beispiel von

a) lokal: Kontaktdermatitis, Ekzemen der verschiedensten Art, Neurodermitis, Erythrodermie, Verbrennungen 1. Grades, Pruritus vulvae et ani, Rosacea, Erythematodes curaneu Psoriasis, Lichen ruber planus et verrucosus;

b) oral: Akute und chronische Polyarthritis, Neurodermitis, Asthma bronchiale, Heufieber u.a.

Darüberhinaus eignen sich die erfindungsgemäßen Kortikoide auch zur Behandlung allergischer Erkrankungen der Atemwege, wie zum beispiel der Rhinitis oder des Bronchialasthmas.

Die Herstellung der Arzneimittelspezialitäten erfolgt in üblicher Weise, indem man die Wirkstoffe mit geeigneten Zusätzen Trägersubstanzen und Geschmackskorrigentien in die gewünschte Applikationsformen wie Tabletten, Dragees, Kapseln, Lösungen, Salben, Inhalationsmitteln usw. überführt.

Für die orale Anwendung eignen sich insbesondere Tabletten, Dragees und Kapseln, welche beispielsweise 0,1—50 mg Kortikoid und 50 mg—2 g eines pharmakologisch unwirksamen Trägers, wie zum Beispiel Laktose, Amylose, Talkum, Gelatine, Magnesiumstearat und ähnliches, sowie die üblichen Zusätze enthalten. Für die topische Anwendung eignen sich Puder, Salben, Aerosole und ähnliche Zubereitungen, die vorzugsweise 0,01 bis 2% des Kortikoids enthalten.

Die nachfolgenden Beispiele dienen zur weiteren Erläuterung der Erfindung.

### Beispiel 1

In eine Lösung von 1.0 g 6$\alpha$-Fluor-11$\beta$-hydroxy-16$\alpha$-methyl-3,20-dioxo-1,4-pregnadien-21-säure-butylester in 25 ml Dimethylformamid wird 30 Stunden ein langsamer Strom von Ammoniak eingeleitet. Die Lösung wird anschließend in Eiswasser eingerührt, der ausgefallene Niederschlag abfiltriert, mit Wasser gewaschen und in Dichlormethan aufgenommen. Die organische Phase wird über Natriumsulfat getrocknet und im Vakuum eingeengt. Der Rückstand wird an 50 g Kieselgel chromatographiert. Mit 33—45% Aceton-Hexan wird das Produkt eluiert und aus Aceton-Benzin (Siedebereich: 60—80°C) umkristallisiert. Ausbeute: 327 mg 6$\alpha$-Fluor-11$\beta$-hydroxy-16$\alpha$-methyl-3,20-dioxo-1,4-pregnadien-21-säure-amid. Schmelzpunkt: 219°C. $[\alpha]_D = +148°$ (Chloroform). UV: $\varepsilon_{241} = 18400$ (Methanol).

### Beispiel 2

2.0 g 6$\alpha$-Fluor-11$\beta$-hydroxy-16$\alpha$-methyl-3,20-dioxo-1,4-pregnadien-21-säure-butylester werden in 30 ml Hexamethylphosphorsäuretriamid gelöst. Es wird eine Stunde lang ein langsamer Strom von Methylamin eingeleitet und wie im Beispiel 1 beschrieben aufgearbeitet und chromatographiert. Das Reaktionsprodukt wird mit 45—60% Aceton-Hexan eluiert und aus Diethylether-Diisopropylether umkristallisiert. Ausbeute: 888 mg 6$\alpha$-Fluor-11$\beta$-hydroxy-16$\alpha$-methyl-3,20-dioxo-1,4-pregnadien-21-säure-methylamid. Schmelzpunkt: 209°C. $[\alpha]_D = +144°$ (Chloroform). UV: $\varepsilon_{242} = 19000$ (Methanol).

### Beispiel 3

Die Lösung von 1.5 g 6$\alpha$-Fluor-11$\beta$-hydroxy-16$\alpha$-methyl-3,20-dioxo-1,4-pregnadien-21-säure-butylester in einem Gemisch aus 10 ml Hexamethylphosphorsäuretriamid und 10 ml Ethylamin wird 1 Stunde bei Raumtemperatur gerührt und wie im Beispiel 1 beschrieben aufgearbeitet und chromatographiert. Mit 25—32% Aceton-Hexan erhält man, nach dem Umkristallisieren aus Aceton-Benzin (Siedebereich 60—80°C), 890 mg 6$\alpha$-Fluor-11$\beta$-hydroxy-16$\alpha$-methyl-3,20-dioxo-1,4-pregnadien-21-säure-ethylamid. Schmelzpunkt: 173°C. $[\alpha]_D = +136°$ (Chloroform).
UV: $\varepsilon_{242} = 18200$ (Methanol).

### Beispiel 4

Eine Lösung von 5.0 g 6$\alpha$-Fluor-11$\beta$-hydroxy-16$\alpha$-methyl-3,20-dioxo-1,4-pregnadien-21-säure-butylester in 10 ml Butylamin läßt man zwei Stunden bei Raumtemperatur stehen. Anschließend wird die Lösung in essigsäurehaltiges Wasser eingerührt, der Niederschlag abfiltriert und in Dichlormethan aufgenommen. Die organische Phase wird mit Wasser gewaschen, über Natriumsulfat getrocknet und im Vakuum eingeengt. Der Rückstand wird an 250 g Kieselgel chromatographiert. Mit 18—22% Aceton-Hexan erhält man, nach dem Umkristallisieren aus Aceton-Benzin (Siedebereich 60—80°C), 4.11 g 6$\alpha$-Fluor-11$\beta$-hydroxy-16$\alpha$-methyl-3,20-dioxo-1,4-pregnadien-21-säure-butylamid. Schmelz-

punkt: 225°C. $[\alpha]_D = +143°$ (Chloroform): UV: $\varepsilon_{242} = 19800$ (Methanol).

Beispiel 5

Eine Lösung von 1.5 g 6α-Fluor-11β-hydroxy-16α-methyl-3,20-dioxo-1,4-pregnadien-21-säure-butylester in 10 ml Dimethylformamid und 5 ml Pentylamin wird zwei Stunden bei Raumtemperatur gerührt. Es wird wie im Beispiel 1 beschrieben aufgearbeitet und chromatographiert. Mit 24—30% Aceton-Hexan erhält man, nach dem Umkristallisieren aus Diethylether-Benzin (Siedebereich 60—80°C), 940 mg 6α-Fluor-11β-hydroxy-16α-methyl-3,20-dioxo-1,4-pregnadien-21-säure-pentylamid. Schmelzpunkt: 170°C. $[\alpha]_D = +140°$ (Chloroform). UV: $\varepsilon_{242} = 19600$ (Methanol).

Beispiel 6

2.0 g 6α-Fluor-11β-hydroxy-16α-methyl-3,20-dioxo-1,4-pregnadien-21-säure-butylester werden in einem Gemisch aus 10 ml Hexamethylphosphorsäuretriamid und 10 ml Hexylamin 90 Minuten bei Raumtemperatur gerührt. Das Reaktionsprodukt wird, wie im Beispiel 1 beschrieben, isoliert und chromatographiert. Mit 20—25% Aceton-Hexan erhält man, nach dem Umkristallisieren aus Aceton-Benzin (Siedebereich 60—80°C) 1.57 g 6α-Fluor-11β-hydroxy-16α-methyl-3,20-dioxo-1,4-pregnadien-21-säure-hexylamid. Schmelzpunkt: 189°C. $[\alpha]_D = +133°$ (Chloroform). UV: $\varepsilon_{242} = 19600$ (Methanol).

Beispiel 7

2.0 g 6α-Fluor-11β-hydroxy-16α-methyl-3,20-dioxo-1,4-pregnadien-21-säure-butylester werden in einem Gemisch aus 10 ml Dimethylformamid und 10 ml Cyclohexylamin zwei Stunden bei Raumtemperatur gerührt. Das Reaktionsprodukt wird, wie im Beispiel 1 beschrieben isoliert und chromatographiert. Mit 25—31% Aceton-Hexan erhält, nach dem Umkristallisieren aus Aceton-Benzin (Siedebereich 60—80°C). 1.31 g 6α-Fluor-11β-hydroxy-16α-methyl-3,20-dioxo-1,4-pregnadien-21-säure-cyclohexylamid. Schmelzpunkt: 257°C. $[\alpha]_D = +142°$ (Chloroform). UV: $\varepsilon_{243} = 19500$ (Methanol).

Beispiel 8

Eine Lösung von 1.0 g 6α-Fluor-11β-hydroxy-16α-methyl-3,20-dioxo-1,4-pregnadien-21-säure-butylester in 25 ml Piperidin wird 3 Stunden bei Raumtemperatur gerührt. Das Reaktionsprodukt wird, wie im Beispiel 1 beschrieben, isoliert und chromatographiert. Mit 20—27% Aceton-Hexan erhält man, nach dem Umkristallisieren aus Diethylester-Diisopropylether, 598 mg 6α-Fluor-11β-hydroxy-16α-methyl-3,20-dioxo-1,4-pregnadien-21-säure-piperidid. Schmelzpunkt: 184°C. $[\alpha]_D = +117°$ (Chloroform). $\varepsilon_{242} = 18700$ (Methanol).

Beispiel 9

Eine Lösung von 800 mg 6α-Fluor-11β-hydroxy-16α-methyl-3,20-dioxo-1,4-pregnadien-21-säure-butylester in 20 ml Morpholin wird 40 Stunden auf 60°C erhitzt. Das Morpholin wird anschließend im Vakuum bei 60°C abdestilliert und der Rückstand an 50 g Kieselgel chromatographiert. Mit 30—36% Aceton-Hexan erhält man, nach dem Umkristallisieren aus Aceton-Diisopropylether, 618 mg 6α-Fluor-11β-hydroxy-16α-methyl-3,20-dioxo-1,4-pregnadien-21-säure-morpholid. Schmelzpunkt: 106°C. $[\alpha]_D = +111°$ (Chloroform). UV: $\varepsilon_{242} = 16500$ (Methanol).

Beispiel 10

Eine Lösung von 1.17 g 6α-Fluor-11β-hydroxy-16α-methyl-3,20-dioxo-1,4-pregnadien-21-säure in 30 ml Tetrahydrofuran wird auf −60°C gekühlt und mit 1 ml Triäthylamin versetzt. Zu dieser Lösung läßt man langsam eine Lösung von 0.43 ml Chlorameisensäureisobutylester in 10 ml Tetrahydrofuran tropfen. Nach 30 Minuten versetzt man mit 1 ml Diethylamin und läßt die Temperatur langsam auf 20°C ansteigen. Nach einer Stunde wird mit Wasser versetzt, mit verdünnter Salzsäure angesäuert und mit Dichlormethan extrahiert. Die organische Phase wird mit Wasser gewaschen, über Natriumsulfat getrocknet und im Vakuum eingedampft. Der Rückstand wird an 100 g Kieselgel chromatographiert. Mit 35—41% Aceton-Hexan erhält man, nach dem Umkristallisieren aus Aceton-Hexan, 290 mg 6α-Fluor-11β-hydroxy-16α-methyl-3,20-dioxo-1,4-pregnadien-21-säure-diethylamid. Schmelzpunkt: 130°C. $[\alpha]_D = +120°$ (Chloroform). UV: $\varepsilon_{242} = 18200$ (Methanol).

Beispiel 11

1.17 g 6α-Fluor-11β-hydroxy-16α-methyl-3,20-dioxo-1,4-pregnadien-21-säure werden, wie im Beispiel 10 beschrieben, jedoch mit 1 ml Benzylamin anstelle von Diethylamin zur Reaktion gebracht. Es wird analog aufgearbeitet und an 100 g Kieselgel chromatographiert. Mit 37—43% Aceton-Hexan erhält man, nach dem Umkristallisieren aus Diethyläther, 800 mg 6α-Fluor-11β-hydroxy-16α-methyl-3,20-dioxo-1,4-pregnadien-21-säure-benzylamid. Schmelzpunkt: 188°C. $[\alpha]_D = +142°C$. (Chloroform). UV: $\varepsilon_{242} = 20100$ (Methanol).

Beispiel 12

1.0 g 6$\alpha$-Fluor-11$\beta$-hydroxy-16$\alpha$-methyl-3,20-dioxo-1,4-pregnadien-21-säure werden, wie im Beispiel 10 beschrieben, jedoch mit 2-Butylamin anstelle von Diethylamin, zur Reaktion gebracht. Es wird entsprechend aufgearbeitet und an 100 g Kieselgel chromatographiert. Mit 17—19% Aceton-Hexan erhält man, nach dem Umkristallisieren aus Aceton-Hexan, 388 mg 6$\alpha$-Fluor-11$\beta$-hydroxy-16$\alpha$-methyl-3,20-dioxo-1,4-pregnadien-21-säure-2-butylamid. Schmelzpunkt 233°C. $[\alpha]_D = +142°$ (Chloroform). UV: $\varepsilon_{242} = 19100$ (Methanol).

Beispiel 13

In eine Lösung von 2.5 g 6$\alpha$-Fluor-11$\beta$-hydroxy-16$\alpha$-methyl-3,20-dioxo-1,4-pregnadien-21-säure-butylester in 25 ml Dimethylformamid wird eine Stunde Dimethylamin eingeleitet. Man läßt die Reaktionsmischung 15 Stunden bei Raumtemperatur stehen, gießt dann in Eiswasser und extrahiert mit Dichlormethan. Der Extrakt wird mit Wasser gewaschen, über Natriumsulfat getrocknet und im Vakuum eingeengt. Der Rückstand wird an Kieselgel chromatographiert. Mit 24—35% Aceton-Dichlormethan erhält man, nach dem Umkristallisieren aus Aceton-Diisopropylether, 105 g 6$\alpha$-Fluor-11$\beta$-hydroxy-16$\alpha$-methyl-3,20-dioxo-1,4-pregnadien-21-säure-dimethylamid. Schmelzpunkt: 236°C. $[\alpha]_D = +161°$ (Chloroform). UV: $\varepsilon_{241} = 18300$ (Methanol).

Beispiel 14

In eine Lösung von 1.45 g 6$\alpha$-Fluor-11$\beta$-hydroxy-16$\alpha$-methyl-3,20-dioxo-1,4-pregnadien-21-säure in 50 ml Tetrahydrofuran wird nach Zugabe von 2.15 g N-Ethoxycarbonyl-2-ethoxy-1,2-dihydrochinol in 30 Minuten bei Raumtempertur Dimethylamin eingeleitet. Das Reaktionsgemisch wird 15 Stunden bei Raumtemperatur stehen gelassen und das Lösungsmittel anschließend im Vakuum verdampft. Der Rückstand wird in Dichlormethan gelöst, die Lösung wird mit Wasser gewaschen, über Natriumsulfat getrocknet und im Vakuum eingedampft. Der Rückstand wird an 200 g Kieselgel chromatographiert. Mit 58—69% Aceton-Hexan erhält man, nach dem Umkristallisieren aus Aceton-Diisopropylether, 1.04 g 6$\alpha$-Fluor-11$\beta$-hydroxy-16$\alpha$-methyl-3,20-dioxo-1,4-pregnadien-21-säure-dimethylamid. Schmelzpunkt: 238°C. $[\alpha]_D = +161°$ (Chloroform). UV: $\varepsilon_{241} = 18000$ (Methanol).

Beispiel 15

Die Lösung von 1.0 g 6$\alpha$-Fluor-11$\beta$-hydroxy-16$\alpha$-methyl-3,20-dioxo-4-pregnen-21-säure-butylester in 10 ml Hexamethylphosphorsäuretriamid wird mit 10 ml Ethylamin versetzt und 4 Stunden bei Raumtemperatur stehen gelassen. Das Reaktionsprodukt wird, wie im Beispiel 1 beschrieben, isoliert und chromatographiert. Mit 42—57% Essigester-Hexan erhält man, nach dem Umkristallisieren aus Ether-Diisopropylether, 436 mg 6$\alpha$-Fluor-11$\beta$-hydroxy-16$\alpha$-methyl-3,20-dioxo-1,4-pregnen-21-säure-ethylamid. Schmelzpunkt: 168°C. $[\alpha]_D = +178°$ (Chloroform). UV: $\varepsilon_{237} = 18400$ (Methanol).

Beispiel 16

Eine Lösung von 3.0 g 6$\alpha$-Fluor-16$\alpha$-methyl-3.11.20-trioxo-1,4-pregnadien-21-säure-butylester in 15 ml Butylamin wird eine Stunde bei Raumtemperatur stehen gelassen und, wie im Beispiel 1 beschrieben, aufgearbeitet. Das Rohprodukt wird aus Aceton-Benzin (Siedebereich 60—80°C) umkristallisiert. Ausbeute: 2.86 g 6$\alpha$-Fluor-16$\alpha$-methyl-3,11,20-trioxo-1,4-pregnadien-21-säure-butylamid. Schmelzpunkt: 185°C. $[\alpha]_D = +198°$ (Chloroform). UV: $\varepsilon_{238} = 19100$ (Methanol).

Beispiel 17

In die Lösung von 6$\alpha$-Fluor-16$\alpha$-methyl-3,11,20-trioxo-1,4-pregnadien-21-säure in 10 ml Hexamethylphosphorsäuretriamid werden bei —15° 2 ml Thionylchlorid eingetropft. Man erwärmt langsam auf Raumtemperatur, leitet 30 Minuten Dimethylamin ein und gießt nach 2 Stunden in Eiswasser. Das ausgefällte Produkt wird abfiltriert, mit Wasser gewaschen, getrocknet und an 50 g Kieselgel chromatographiert. Mit 21—23% Aceton-Hexan erhält man, nach dem Umkristallisieren aus Aceton-Diisopropylether, 350 mg 6$\alpha$-Fluor-16$\alpha$-methyl-3,11,20-trioxo-1,4-pregnadien-21-säure-dimethylamid. Schmelzpunkt: 175°C. $[\alpha]_D = +182°$ (Chloroform). UV: $\varepsilon_{238} = 17600$ (Methanol).

Beispiel 18

2.0 g 6$\alpha$,9$\alpha$ - Difluor - 11$\beta$ - hydroxy - 16$\alpha$ - methyl - 3,20 - dioxo - 1,4 - pregnadien - 21 - säure-butylester löst man in 30 ml Dimethylformamid und leitet bei Raumtemperatur 20 Stunden Ammoniak ein. Das Reaktionsprodukt wird mit Eiswasser gefällt, abfiltriert, getrocknet und aus Essigester umkristallisiert. Ausbeute: 1.53 g 6$\alpha$,9$\alpha$-Difluor-11$\beta$-dihydroxy-16$\alpha$-methyl-3.20-dioxo-1,4-pregnadien-21-säure-amid. Schmelzpunkt: >300°C. $[\alpha]_D = +213°$ (Pyridin). UV: $\varepsilon_{237} = 19000$ (Methanol).

Beispiel 19

In eine Lösung von 5.0 g 6$\alpha$,9$\alpha$-Difluor-11$\beta$-hydroxy-16$\alpha$-methyl-3,20-dioxo-1,4-pregnadien-21-säure-methylester in 60 ml Hexamethylphosphorsäuretriamid wird 30 Minuten Methylamin eingeleitet. Anschließend läßt man die Lösung eine Stunde bei Raumtemperatur stehen und gießt in Eiswasser. Das ausgefällte Produkt wird abfiltriert, mit Wasser gewaschen, an der Luft getrocknet und aus

Aceton-Diisopropylether umkristallisiert. Ausbeute: 3.51 g 6α,9α-Difluor-11β-hydroxy-16α-methyl-3,20-dioxo-1,4-pregnadien-21-säure-methylamid. Schmelzpunkt: 234°C. $[\alpha]_D = +131°$ (Chloroform). UV: $\varepsilon_{238} = 19500$ (Methanol).

Beispiel 20

Eine Lösung von 300 mg 6α,9α-Difluor-11β-hydroxy-16α-methyl-3,20-dioxo-1,4-pregnadien-21-säure in 33 ml Hexamethylphosphorsäuretriamid wird auf —15°C gekühlt. Man setzt 0.54 ml Thionylchlorid hinzu, leitet 15 Minuten Dimethylamin ein und läßt anschließend 5 Stunden bei Raumtemperatur stehen. Das Reaktionsgemisch wird in Eiswasser gegossen, der ausgefallene Niederschlag wird abfiltriert, mit Wasser gewaschen und getrocknet. Das Rohprodukt wird mittels präparativer Schichtchromatographie an Kieselgel gereinigt (Laufmittel: 50% Hexan-Essigester) und aus Aceton-Diisopropylether umkristallisiert. Ausbeute: 164 mg 6α,9α-Difluor-11β-hydroxy-16α-methyl-3,20-dioxo-1,4-pregnadien-21-säure-dimethylamid. Schmelzpunkt 269°C. $[\alpha]_D = +108°$ (Chloroform). UV: $\varepsilon_{238} = 15200$ (Methanol).

Beispiel 21

Eine Lösung von 600 mg 6α,9α-Difluor-11β-hydroxy-16α-methyl-3,20-dioxo-1,4-pregnadien-21-säure in 6 ml Hexamethylphosphorsäuretriamid wird auf —15°C gekühlt und mit 1.1 ml Thionylchlorid sowie 1 ml Diethylamin versetzt. Die Lösung wird 5 Stunden bei Raumtemperatur gerührt und anschließend in Eiswasser eingegossen. Der ausgefallene Niederschlag wird abfiltriert, mit Wasser gewaschen, getrocknet und mittels präparativer Schichtchromatographie an Kieselgel gereinigt (Laufmittel: 50% Hexan-Essigester). Das Produkt wird aus Aceton-Diisopropylether umkristallisiert. Ausbeute: 298 mg 6α,9α - Difluor - 11β - hydroxy - 16α - methyl - 3,20 - dioxo - 1,4 - pregnadien - 21 - säure - diethylamid. Schmelzpunkt: 158°C. $[\alpha]_D = +109°$ (Chloroform). UV: $\varepsilon_{238} = 18700$ (Methanol).

Beispiel 22

1.30 g 9α-Chlor-6α-fluor-11β-hydroxy-16α-methyl-3,20-dioxo-1,4-pregnadien-21-säure-butylester werden analog Beispiel 18 in 9α-Chlor-6α-fluor-11β-hydroxy-16α-methyl-3,20-dioxo-1,4-pregnadien-21-säure-amid überführt. Ausbeute: 920 mg, umkristallisiert aus Aceton-Diisopropylether. Schmelzpunkt: 238°C. $[\alpha]_D = +223°$ (Pyridin). UV: $\varepsilon_{237} = 18300$ (Methanol).

Beispiel 23

In eine Lösung von 2.0 g 9α-Chlor-6α-fluor-11β-hydroxy-16α-methyl-3,20-dioxo-1,4-pregnatrien-21-säure-butylester in 30 ml Hexamethylphosphorsäuretriamid leitet man 2 Stunden Methylamin ein und fällt das Reaktionsprodukt mit Eiswasser. Der Niederschlag wird abfiltriert, gewaschen, getrocknet und an 100 g Kieselgel chromatographiert. Mit 53—67% Aceton-Hexan erhält man, nach dem Umkristallisieren aus Aceton-Diisopropylether, 920 mg 9α-Chlor-6α-fluor-11β-hydroxy - 16α - methyl - 3,20 - dioxo - 1,4 - pregnadien - 21 - säure - methylamid. Schmelzpunkt: 230°C. $[\alpha]_D = +203°$ (Pyridin). UV: $\varepsilon_{238} = 18400$ (Methanol).

Beispiel 24

Eine Lösung von 1.0 g 9α-Chlor-6α-fluor-11β-hydroxy-16α-methyl-3,20-dioxo-1,4-pregnadien-21-säure-methylester in 6 ml Morpholin wird 3 Stunden auf 60°C erhitzt. Das Morpholin wird anschließend im Vakuum abdestilliert und der Rückstand an 50 g Kieselgel chromatographiert. Mit 26—35% Aceton-Hexan erhält man, nach dem Umkristallisieren aus Essigester-Diisopropylether, 867 mg 9α - Chlor - 6α - fluor - 11β - hydroxy - 16α - methyl - 3,20 - dioxo - 1,4 - pregnadien - 21 - säure-morpholid. Schmelzpunkt: 216°C. $[\alpha]_D = +132°$ (Chloroform). UV: $\varepsilon_{238} = 18100$ (Methanol).

Beispiel 25

a) Eine Loßung von 500 mg 6α-Fluor-11β-hydroxy-16α-methyl-3,20-dioxo-1,4-pregnadien-21-säure in 5 ml Hexamethylphosphorsäuretriamid wird auf —15°C gekühlt und mit 0.9 ml Thionylchlorid versetzt. Man erwärmt langsam auf Raumtemperatur und rührt nach 6 Stunden in Eiswasser ein. Der Niederschlag wird avfiltriert, gewaschen, getrocknet und an 50 g Kieselgel chromatographiert. Mit 27—34% Aceton-Hexan erhält man, nach dem Umkristallisieren aus Diethylether, 315 mg 6α-Fluor-16α-methyl-3,20-dioxo-1,4,9(11)-pregnatrien-21-säure-dimethylamid. Schmelzpunkt: 136°C. $[\alpha]_D = +49°$ (Chloroform). UV: $\varepsilon_{238} = 18500$ (Methanol).

b) Die Lösung von 150 mg 6α-Fluor-16α-methyl-3,20-dioxo-1,4,9(11)-pregnatrien-21-säure-dimethylamid in 5.5 ml Dioxan wird mit 1.4 ml Wasser, 550 mg N-Chlorsuccinimid sowie 0.55 ml 70proz. Perchlorsäure versetzt und 10 Minuten bei Raumtemperatur gerührt. Das Reaktionsgemisch wird in Eiswasser gegossen, der ausgefallene Niederschlag abfiltriert, mit Wasser gewaschen und an der Luft getrocknet. Nach dem Umkristallisieren aus Aceton-Diisopropylether erhältman 109 mg 9α-Chlor-6α-fluor-11β-hydroxy-16α-methyl-3,20-dioxo-1,4-pregnadien-21-säure-dimethylamid. Schmelzpunkt: 228°C. $[\alpha]_D = +169°$ (Chloroform). UV: $\varepsilon_{238} = 18100$ (Methanol).

Beispiel 26

a) Eine auf —15°C gekühlte Lösung von 3.0 g 6α-Fluor-11β-hydroxy-16α-methyl-3,20-dioxo-1,4-pregnadien-21-säure in 30 ml Hexamethylphosphorsäuretriamid wird mit 5.4 ml Thionylchlorid und 6.9 ml Diethylamin versetzt. Anschließend läßt man das Reaktionsgemisch 5 Stunden bei Raumtemperatur stehen und fällt mit Eiswasser. Der Niederschlag wird abfiltriert, mit Wasser gewaschen, getrocknet und an 200 g Kieselgel chromatographiert. Mit 22—26% Aceton-Hexan erhält man, nach dem Umkristallisieren aus Aceton-Hexan, 1.50 g 6α-Fluor-16α-methyl-3,20-dioxo-1,4,9(11)-pregnatrien-21-säure-diethylamid. Schmelzpunkt: 144°C. $[\alpha]_D = +59°$ (Chloroform). UV: $\varepsilon_{238} = 18400$ (Methanol).

b) 200 mg 6α-Fluor-16α-methyl-3,20-dioxo-1,4,9(11)-pregnatrien-21-säure-diethylamid werden in 7.4 ml Dioxan gelöst. Die Lösung wird mit 1.85 ml Wasser, 750 mg N-Chlorsuccinimid sowie 0.75 ml 70proz. Perchlorsäure versetzt, 10 Minuten bei Raumtemperatur gerührt und in Eiswasser gegossen. Der ausgefallene Niederschlag wird abfiltriert, mit Wasser gewaschen, an der Luft getrocknet und aus Aceton-Diisopropylether umkristallisiert. Ausbeute: 155 mg 9α-Chlor-6α-fluor-11β-hydroxy-16α-methyl-3,20-dioxo-1,4-pregnadien-21-säure-diethylamid. Schmelzpunkt: 198°C. $[\alpha]_D = +165°$ (Chloroform). UV: $\varepsilon_{238} = 17900$ (Methanol).

Beispiel 27

Eine Lösung von 1.0 g 2-Chlor-6α-fluor-11β-hydroxy-16α-methyl-3,20-dioxo-1,4-pregnadien-21-säure-methylester in 10 ml Pentylamin wird 3 Stunden bei Raumtemperatur stehen gelassen und anschließend, wie im Beispiel 1 beschrieben, aufgearbeitet. Das Rohprodukt wird an 50 g Kieselgel chromatographiert. Mit 20—27% Essigester-Hexan erhält man, nach dem Fällen mit Aceton-Diethylester, 131 mg 2-Chlor-6α-fluor-11β-hydroxy-16α-methyl-3,20-dioxo-1,4-pregnadien-21-säure-pentylamid. $[\alpha]_D = +65°$ (Chloroform). UV: $\varepsilon_{249} = 16800$ (Methanol).

Beispiel 28

Eine Lösung von 600 mg 9α-Chlor-6α,11β-difluor-16α-methyl-3,20-dioxo-1,4-pregnadien-21-säure-methylester in 10 ml Cyclohexylamin wird 3 Stunden bei Raumtemperatur stehen gelassen und anschließend, wie im Beispiel 1 beschrieben, aufgearbeitet. Das Reaktionsprodukt wird an 50 g Kieselgel chromatographiert. Mit 41—82% Essigester-Hexan erhält man, nach dem Umkristallisieren aus Essigester-Diisopropyläther, 425 mg 9α-Chlor-6α,11β-difluor-16α-methyl-3,20-dioxo-1,4-pregnadien-21-säure-cyclohexylamid. Schmelzpunkt: 185°C. $[\alpha]_D = +139°$ (Chloroform). UV: $\varepsilon_{237} = 19200$ (Methanol).

Beispiel 29

Eine Lösung von 600 mg 9α,11β-Dichlor-6α-fluor-16α-methyl-3,20-dioxo-1,4-pregnadien-21-säure-methylester in 5 ml Butylamin wird 5 Stunden bei Raumtemperatur stehen gelassen. Man arbeitet auf und chromatographiert, wie im Beispiel 1 beschrieben. Mit 15—21% Aceton-Hexan wird das Reaktionsprodukt eluiert und aus Diethylether-Benzin (Siedebereich 40—60°C) umkristallisiert. Ausbeute: 544 mg 9α,11β-Dichlor-6α-fluor-16α-methyl-3,20-dioxo-1,4-pregnadien-21-säure-butylamid. Schmelzpunkt: 186°C. $[\alpha]_D = +177°$ (Chloroform). UV: $\varepsilon_{237} = 18700$ (Methanol).

Beispiel 30

In eine Lösung von 1.5 g 9α-Fluor-11β-hydroxy-16α-methyl-3,20-dioxo-1,4-pregnadien-21-säure-methylester in 25 ml Dimethylformamid wird 6 Stunden ein langsamer Strom von Ammoniak eingeleitet. Man läßt dann 36 Stunden bei Raumtemperatur stehen und rührt anschließend in Eiswasser ein. Der ausgefallene Niederschlag wird abfiltriert, mit Wasser gewaschen, getrocknet und aus Aceton-Diisopropylether umkristallisiert. Ausbeute: 913 mg 9α-Fluor-11β-hydroxy-16α-methyl-1,4-pregnadien-21-säure-amid Schmelzpunkt: 266°C. $[\alpha]_D = +235°$ (Pyridin). UV: $\varepsilon_{238} = 17900$ (Methanol).

Beispiel 31

Eine Lösung von 1.0 g 9α-Fluor-11β-hydroxy-16α-methyl-3,20-dioxo-1,4-pregnadien-21-säure-methylester in 20 ml Hexylamin läßt man 3 Stunden bei Raumtemperatur stehen. Das Reaktionsprodukt wird isoliert und chromatographiert, wie im Beispiel 1 beschrieben. Mit 18—22% Aceton-Hexan erhält man, nach dem Umkristallisieren aus Aceton-Diisopropylether, 682 mg 9α-Fluor-11β-hydroxy-16α-methyl-3,20-dioxo-1,4-pregnadien-21-säure-hexylamid. Schmelzpunkt: 123°C. $[\alpha]_D = +131°$ (Chloroform). UV: $\varepsilon_{240} = 18600$ (Methanol).

Beispiel 32

Eine Lösung von 2.0 g 11β-Hydroxy-3,20-dioxo-1,4-pregnadien-21-säure-butylester in 10 ml Butylamin wird 30 Minuten bei Raumtemperatur stehen gelassen. Das Rohprodukt wird, wie im Beispiel 1 beschrieben, isoliert und chromatographiert. Mit 24—29% Aceton-Hexan erhält man, nach dem Umkristallisieren aus Diethylether-Diisopropylether, 853 mg 11β-Hydroxy-3,20-dioxo-1,4-

pregnadien-21-säure-butylamid. Schmelzpunkt: 150°C. $[\alpha]_D = +185°$ (Chloroform). UV: $\varepsilon_{243} = 18100$ (Methanol).

Beispiel 33

2.0 g 11β-Hydroxy-3,20-dioxo-1,4-pregnadien-21-säure-butylester versetzt man mit 10 ml Morpholin und erwärmt 4 Stunden auf 60°C. Das Morpholin wird anschließend im Vakuum abdestilliert und der Rückstand an 100 g Kieselgel chromatographiert. Mit 23—30% Aceton-Hexan erhält man, nach dem Umkristallisieren aus Aceton-Benzin (Siedebereich 60—80°C), 1.02 g 11β-Hydroxy-3,20-dioxo-1,4-pregnadien-21-säure-morpholid. Schmelzpunkt: 194°C $[\alpha]_D = +146°$ (Chloroform). UV: $\varepsilon_{243} = 17700$ (Methanol).

Beispiel 34

5.0 g 11β-Hydroxy-3,20-dioxo-1,4-pregnadien-21-säure-butylester werden analog Beispiel 2 in 11β - Hydroxy - 3,20 - dioxo - 1,4 - pregnadien - 21 - säure - methylamid überführt. Ausbeute: 3.30 g. umkristallisiert aus Aceton. Schmelzpunkt: 147°C. $[\alpha]_D = +255°$ (Pyridin). UV: $\varepsilon_{243} = 17500$ (Methanol).

Beispiel 35

5.0 g 11β-Hydroxy-3,20-dioxo-1,4-pregnadien-21-säure-butylester werden analog Beispiel 1 in 11β-Hydroxy-3,20-dioxo-1,4-pregnadien-21-säureamid überführt. Ausbeute: 1.47 g, umkristallisiert aus Aceton-Diisopropylether. Schmelzpunkt: 194°C. $[\alpha]_D = +263°$ (Pyridin). UV: $\varepsilon_{241} = 16600$ (Methanol).

**Patentansprüche**

1. Kortikoide der allgemeinen Formel I

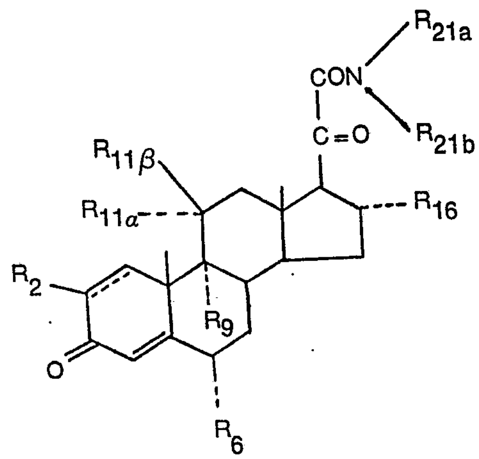

(I),

worin

die Bindung ...... eine Einfachbindung oder eine Doppelbindung,
$R_2$ ein Wasserstoffatom oder ein Chloratom,
$R_6$ ein Wasserstoffatom oder ein Fluoratom und
$R_{16}$ ein Wasserstoffatom oder eine Methylgruppe bedeuten, worin
$R_9$ ein Wasserstoffatom, ein Fluoratom oder ein Chloratom und
$R_{11\alpha}$ und $R_{11\beta}$ gemeinsam eine Oxogruppe oder $R_{11\alpha}$ ein Wasserstoff atom und $R_{11\beta}$ eine Hydroxygruppe oder falls $R_9$ ein Chloratom ist auch ein Fluoratom oder ein Chloratom darstellen und worin $R_{21a}$ und $R_{21b}$ gleich oder verschieden sind und Wasserstoff oder bis zu 8 Kohlenstoffatome enthaltende Alkylreste oder Cycloalkylreste oder gemeinsam einen gegebenenfalls durch Sauerstoff oder Stickstoff unterbrochenen bis zu 8 Kohlenstoffatome enthaltenden Alkylenrest darstellen.

2. 6α-Fluor-11β-hydroxy-16α-methyl-3,20-dioxo-1,4-pregnadien-21-säure-amid.
3. 6α-Fluor-11β-hydroxy-16α-methyl-3,20-dioxo-1,4-pregnadien-21-säure-methylamid.
4. 6α-Fluor-11β-hydroxy-16α-methyl-3,20-dioxo-1,4-pregnadien-21-säure-ethylamid.
5. 6α-Fluor-11β-hydroxy-16α-methyl-3,20-dioxo-1,4-pregnadien-21-säure-butylamid.
6. 6α-Fluor-11β-hydroxy-16α-methyl-3,20-dioxo-1,4-pregnadien-21-säure-pentylamid.
7. 6α-Fluor-11β-hydroxy-16α-methyl-3,20-dioxo-1,4-pregnadien-21-säure-hexylamid.
8. 6α-Fluor-11β-hydroxy-16α-methyl-3,20-dioxo-1,4-pregnadien-21-säure-cyclohexylamid.
9. 6α-Fluor-11β-hydroxy-16α-methyl-3,20-dioxo-1,4-pregnadien-21-säure-piperidid.
10. 6α-Fluor-11β-hydroxy-16α-methyl-3,20-dioxo-1,4-pregnadien-21-säure-morpholid.
11. 6α-Fluor-11β-hydroxy-16α-methyl-3,20-dioxo-1,4-pregnadien-21-säure-diethylamid.

12. 6α-Fluor-11β-hydroxy-16α-methyl-3,20-dioxo-1,4-pregnadien-21-säure-benzylamid.
13. 6α-Fluor-11β-hydroxy-16α-methyl-3,20-dioxo-1,4-pregnadien-21-säure-2-butylamid.
14. 6α-Fluor-11β-hydroxy-16α-methyl-3,20-dioxo-1,4-pregnadien-21-säure-dimethylamid.
15. 6α-Fluor-11β-hydroxy-16α-methyl-3,20-dioxo-1,4-pregnen-21-säure-ethylamid.
16. 6α-Fluor-16α-methyl-3,11,20-trioxo-1,4-pregnadien-21-säure-butylamid.
17. 6α-Fluor-16α-methyl-3,11,20-trioxo-1,4-pregnadien-21-säure-dimethylamid.
18. 6α,9α-Difluor-11β-hydroxy-16α-methyl-3,20-dioxo-1,4-pregnadien-21-säure-amid.
19. 6α,9α-Difluor-11β-hydroxy-16α-methyl-3,20-dioxo-1,4-pregnadien-21-säure-methylamid.
20. 6α,9α-Difluor-11β-hydroxy-16α-methyl-3,20-dioxo-1,4-pregnadien-21-säure-dimethylamid.
21. 6α,9α-Difluor-11β-hydroxy-16α-methyl-3,20-dioxo-1,4-pregnadien-21-säure-diethylamid.
22. 9α-Chlor-6α-fluor-11β-hydroxy-16α-methyl-3,20-dioxo-1,4-pregnadien-21-säure-amid.
23. 9α-Chlor-6α-fluor-11β-hydroxy-16α-methyl-3,20-dioxo-1,4-pregnadien-21-säure-methylamid.
24. 9α - Chlor - 6α - fluor - 11β - hydroxy - 16α - methyl - 3,20 - dioxo - 1,4 - pregnadien - 21 - säure - morpholid.
25. 9α - Chlor - 6α - fluor - 11β - hydroxy - 16α - methyl - 3,20 - dioxo - 1,4 - pregnadien - 21 - säure - dimethylamid.
26. 9α - Chlor - 6α - fluor - 11β - hydroxy - 16α - methyl - 3,20 - dioxo - 1,4 - pregnadien - 21 - säure - diethylamid.
27. 2 - Chlor - 6α - fluor - 11β - hydroxy - 16α - methyl - 3,20 - dioxo - 1,4 - pregnadien - 21 - säure - pentylamid.
28. 9α - Chlor - 6α-,11β - difluor - 16α - methyl - 3,20 - dioxo - 1,4 - pregnadien - 21 - säure-cyclohexylamid.
29. 9α,11β-Dichlor-6α-fluor-16α-methyl-3,20-dioxo-1,4-pregnadien-21-säure-butylamid.
30. 9α-Fluor-11β-hydroxy-16α-methyl-3,20-dioxo-1,4-pregnadien-21-säure-amid.
31. 9α-Fluor-11β-hydroxy-16α-methyl-3,20-dioxo-1,4-pregnadien-21-säure-hexylamid.
32. 11β-Hydroxy-3,20-dioxo-1,4-pregnadien-21-säure-butylamid.
33. 11β-Hydroxy-3,20-dioxo-1,4-pregnadien-21-säure-morpholid.
34. 11β-Hydroxy-3,20-dioxo-1,4-pregnadien-21-säure-methylamid.
35. 11β-Hydroxy-3,20-dioxo-1,4-pregnadien-21-säureamid.
36. Pharmazeutische Präparate enthaltend ein oder mehrere Kortikoide gemäß Anspruch 1 bis 35 als Wirkstoff.
37. Verfahren zur Herstellung von Verbindungen der allgemeinen Formel I

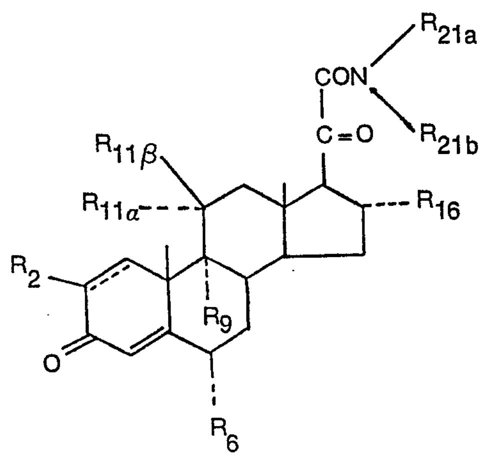

(I),

worin

die Bindung ...... eine Einfachbindung oder eine Doppelbindung,
$R_2$ ein Wasserstoffatom oder ein Chloratom,
$R_6$ ein Wasserstoffatom oder ein Fluoratom und
$R_{16}$ ein Wasserstoffatom oder ein Methylgruppe bedeuten, worin
$R_9$ ein Wasserstoffatom, ein Fluoratom oder ein Chloratom und
$R_{11\alpha}$ und $R_{11\beta}$ gemeinsam eine Oxogruppe oder $R_{11\alpha}$ ein Wasserstoff atom und $R_{11\beta}$ eine Hydroxygruppe oder falls $R_9$ ein Chloratom ist auch ein Fluoratom oder ein Chloratom darstellen und worin $R_{21a}$ und $R_{21b}$ gleich oder verschieden sind und Wasserstoff oder bis zu 8 Kohlenstoffatome enthaltende Alkylreste oder Cycloalkylreste oder gemeinsam einen gegebenenfalls durch Sauerstoff oder Stickstoff unterbrochenen bis zu 8 Kohlenstoffatome enthaltenden Alkylenrest darstellen, dadurch gekennzeichnet, daß man in an sich bekannter Weise

a) eine Carbonsäure der allgemeinen Formel II

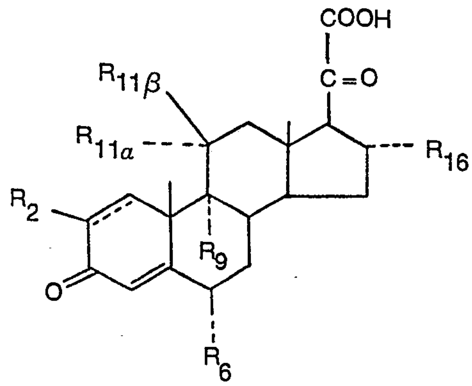

(II),

worin . . . . ., $R_2$, $R_6$, $R_9$, $R_{11\alpha}$, $R_{11\beta}$ und $R_{16}$ die obengenannte Bedeutung besitzen, oder ein reaktionsfähiges Derivat dieser Säure mit einem Amin der allgemeinen Formel III

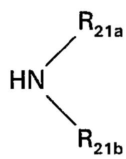

(III),

worin $R_{21a}$ und $R_{21b}$ die obengenannte Bedeutung besitzen, kondensiert, oder

b) zur Herstellung von Kortikoiden mit $R_9$ in der Bedeutung eines Chloratoms an die 9(11)-Doppelbindung einer Verbindung der allgemeinen Formel IV

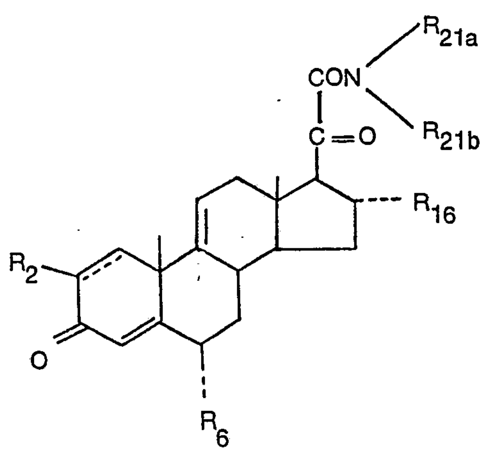

(IV),

worin $R_2$, $R_6$, $R_{16}$, $R_{21a}$ und $R_{21b}$ die obengenannte Bedeutung besitzen, unterchlorige Säure, Fluorchlorid oder Chlor anlagert.

c) Zur Herstellung von Kortikoiden mit $R_{11\beta}$ in der Bedeutung einer Hydroxygruppe und $R_9$ in der Bedeutung eines Fluoratoms oder eines Chloratoms den 9,11-Epoxydring einer Verbindung der allgemeinen Formel V

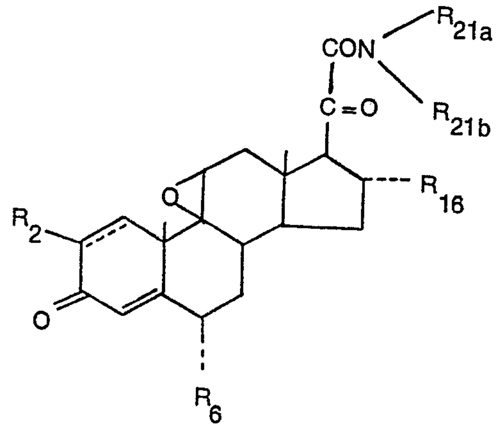

(V),

mit Fluorwasserstoff oder Chlorwasserstoff öffnet.

## Claims

1. Corticoids of the general formula I

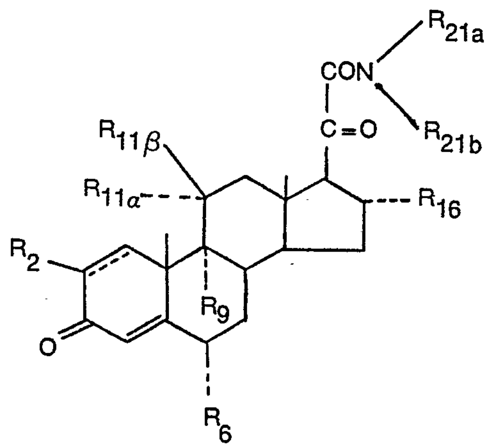

(I),

in which

the bond ........ represents a single bond or a double bond,

$R_2$ represents a hydrogen atom or a chlorine atom,

$R_6$ represents a hydrogen atom or a fluorine atom,

$R_{16}$ represents a hydrogen atom or a methyl group,

$R_9$ represents a hydrogen atom, a fluorine atom or a chlorine atom,

$R_{11\alpha}$ und $R_{11\beta}$ together represent an oxo group or $R_{11\alpha}$ represents a hydrogen atom and $R_{11\beta}$ represents a hydroxy group or, if $R_9$ is a chlorine atom, alternatively a fluorine atom or a chlorine atom, and

$R_{21a}$ and $R_{21b}$ are the same or different and represent hydrogen or alkyl or cycloalkyl radicals containing up to 8 carbon atoms or together represent an alkylene radical that contains up to 8 carbon atoms and that is optionally interrupted by oxygen or nitrogen.

2. 6α-Fluoro-11β-hydroxy-16α-methyl-3,20-dioxo-1,4-pregnadien-21-oic acid amide.

3. 6α-Fluoro-11β-hydroxy-16α-methyl-3,20-dioxo-1,4-pregnadien-21-oic acid methylamide.

4. 6α-Fluoro-11β-hydroxy-16α-methyl-3,20-dioxo-1,4-pregnadien-21-oic acid ethylamide.

5. 6α-Fluoro-11β-hydroxy-16α-methyl-3,20-dioxo-1,4-pregnadien-21-oic acid butylamide.

6. 6α-Fluoro-11β-hydroxy-16α-methyl-3,20-dioxo-1,4-pregnadien-21-oic acid pentylamide.

7. 6α-Fluoro-11β-hydroxy-16α-methyl-3,20-dioxo-1,4-pregnadien-21-oic acid hexylamide.

8. 6α-Fluoro-11β-hydroxy-16α-methyl-3,20-dioxo-1,4-pregnadien-21-oic acid cyclohexylamide.

9. 6α-Fluoro-11β-hydroxy-16α-methyl-3,20-dioxo-1,4-pregnadien-21-oic acid piperidide.

10. 6α-Fluoro-11β-hydroxy-16α-methyl-3,20-dioxo-1,4-pregnadien-21-oic acid morpholide.

11. 6α-Fluoro-11β-hydroxy-16α-methyl-3,20-dioxo-1,4-pregnadien-21-oic acid diethylamide.

12. 6α-Fluoro-11β-hydroxy-16α-methyl-3,20-dioxo-1,4-pregnadien-21-oic acid benzylamide.

13. 6α-Fluoro-11β-hydroxy-16α-methyl-3,20-dioxo-1,4-pregnadien-21-oic acid 2-butylamide.

14. 6α-Fluoro-11β-hydroxy-16α-methyl-3,20-dioxo-1,4-pregnadien-21-oic acid dimethylamide.

15. 6α-Fluoro-11β-hydroxy-16α-methyl-3,20-dioxo-1,4-pregnen-21-oic acid ethylamide.

16. 6α-Fluoro-16α-methyl-3,11,20-trioxo-1,4-pregnadien-21-oic acid butylamide.
17. 6α-Fluoro-16α-methyl-3,11,20-trioxo-1,4-pregnadien-21-oic acid dimethylamide.
18. 6α,9α-Difluoro-11β-hydroxy-16α-methyl-3,20-dioxo-1,4-pregnadien-21-oic acid amide.
19. 6α,9α-Difluoro-11β-hydroxy-16α-methyl-3,20-dioxo-1,4-pregnadien-21-oic acid methylamide.
20. 6α,9α-Difluoro-11β-hydroxy-16α-methyl-3,20-dioxo-1,4-pregnadien-21-oic acid dimethylamide.
21. 6α,9α-Difluoro-11β-hydroxy-16α-methyl-3,20-dioxo-1,4-pregnadien-21-oic acid diethylamide.
22. 9α-Chloro-6α-fluoro-11β-hydroxy-16α-methyl-3,20-dioxo-1,4-pregnadien-21-oic acid amide.
23. 9α-Chloro-6α-fluoro-11β-hydroxy-16α-methyl-3,20-dioxo-1,4-pregnadien-21-oic acid methylamide.
24. 9α-Chloro-6α-fluoro-11β-hydroxy-16α-methyl-3,20-dioxo-1,4-pregnadien-21-oic acid morpholide.
25. 9α-Chloro-6α-fluoro-11β-hydroxy-16α-methyl-3,20-dioxo-1,4-pregnadien-21-oic acid dimethylamide.
26. 9α-Chloro-6α-fluoro-11β-hydroxy-16α-methyl-3,20-dioxo-1,4-pregnadien-21-oic acid diethylamide.
27. 2-Chloro-6α-fluoro-11β-hydroxy-16α-methyl-3,20-dioxo-1,4-pregnadien-21-oic acid pentylamide.
28. 9α-Chloro-6α,11β-difluoro-16α-methyl-3,20-dioxo-1,4-pregnadien-21-oic acid cyclohexylamide.
29. 9α,11β-Dichloro-6α-fluoro-16α-methyl-3,20-dioxo-1,4-pregnadien-21-oic acid butylamide.
30. 9α-Fluoro-11β-hydroxy-16α-methyl-3,20-dioxo-1,4-pregnadien-21-oic acid amide.
31. 9α-Fluoro-11β-hydroxy-16α-methyl-3,20-dioxo-1,4-pregnadien-21-oic acid hexylamide.
32. 11β-Hydroxy-3,20-dioxo-1,4-pregnadien-21-oic acid butylamine.
33. 11β-Hydroxy-3,20-dioxo-1,4-pregnadien-21-oic acid morpholide.
34. 11β-Hydroxy-3,20-dioxo-1,4-pregnadien-21-oic acid methylamide.
35. 11β-Hydroxy-3,20-dioxo-1,4-pregnadien-21-oic acid amide.
36. Pharmaceutical preparations containing as active ingredient one or more corticoids according to claims 1 to 35.
37. Process for the manufacture of compounds of the general formula I

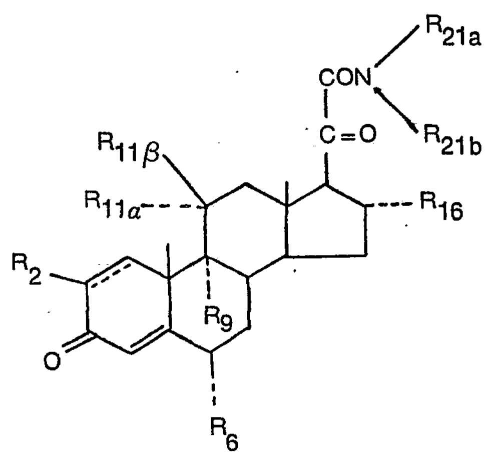

(I),

in which

the bond ....... represents a single bond or a double bond,

$R_2$ represents a hydrogen atom or a chlorine atom,

$R_6$ represents a hydrogen atom or a fluorine atom,

$R_{16}$ represents a hydrogen atom or a methyl group,

$R_9$ represents a hydrogen atom, a fluorine atom or a chlorine atom,

$R_{11\alpha}$ and $R_{11\beta}$ together represent an oxo group or $R_{11\alpha}$ represents a hydrogen atom and $R_{11\beta}$ represents a hydroxy group or, if $R_9$ is a chlorine atom, alternatively a fluorine atom or a chlorine atom, and

$R_{21a}$ and $R_{21b}$ are the same or different and represent hydrogen or alkyl or cycloalkyl radicals containing up to 8 carbon atoms or together represent an alkylene radical that contains up to 8 carbon atoms and that is optionally interrupted by oxygen or nitrogen, characterised in that, in a manner known *per se*,

a) a carboxylic acid of the general formula II

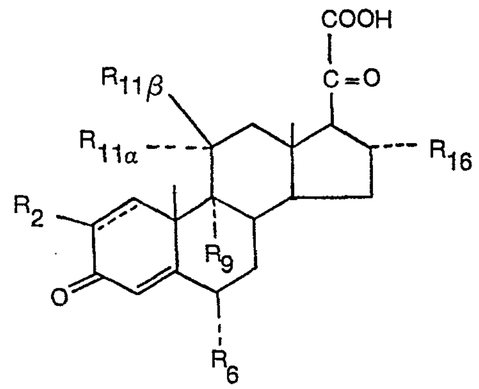

(II),

in which ........, $R_2$, $R_6$, $R_9$, $R_{11\alpha}$, $R_{11\beta}$ and $R_{16}$ have the meanings given above, or a reactive derivative of this acid, is condensed with an amine of the general formula III

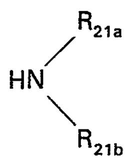

(III),

in which $R_{21a}$ and $R_{21b}$ have the meanings given above, or

b) for the manufacture of corticoids in which $R_9$ represents a chlorine atom, hypochlorous acid, fluorchloride or chlorine is added to the 9(11)-double bond of a compound of the general formula IV

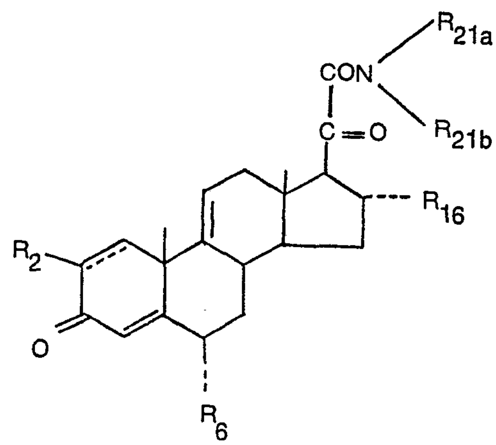

(IV),

in which $R_2$, $R_6$, $R_{16}$, $R_{21a}$ and $R_{21b}$ have the meanings given above, or

c) for the manufacture of corticoids in which $R_{11\beta}$ represents a hydroxy group and $R_9$ represents a fluorine atom or a chlorine atom, the 9,11-epoxide ring of a compound of the general formula V

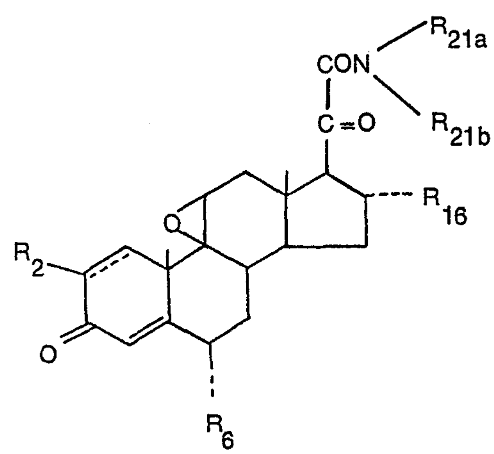

(V),

is opened with hydrogen fluoride or hydrogen chloride.

**Revendications**

1. Corticoïdes répondant à la formule générale I

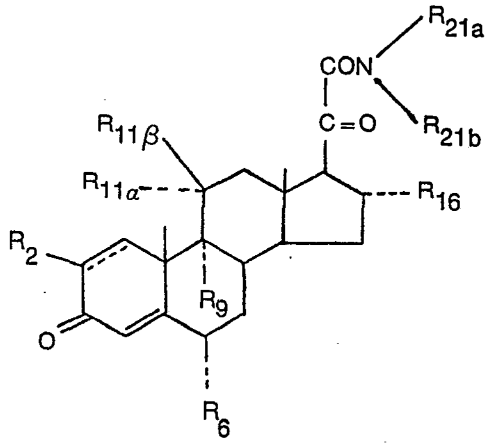

(I),

dans laquelle

la liaison ... est une liaison simple ou une liaison double,

$R_2$ représente un atome d'hydrogène ou de chlore,

$R_6$ représente un atome d'hydrogène ou de fluor,

$R_{16}$ représente un atome d'hydrogène ou un radical méthyle,

$R_9$ représente un atome d'hydrogène, de fluor ou de chlore,

$R_{11\alpha}$ et $R_{11\beta}$ forment ensemble un groupe oxo, ou $R_{11\alpha}$ représente un atome d'hydrogène et $R_{11\beta}$ un groupe hydroxy, ou, lorsque $R_9$ est un atome de chlore, $R_{11\beta}$ est également un atome de fluor ou de chlore, et

$R_{21a}$ et $R_{21b}$ représentent chacun, indépendamment l'un de l'autre, un atome d'hydrogène, un radical alkyle contenant au plus 8 atomes de carbone ou un radical cyclo-alkyle, ou forment ensemble un radical alkylène contenant au plus 8 atomes de carbone et éventuellement interrompu par de l'oxygène ou de l'azote.

2. Amide de l'acide fluoro-6$\alpha$ hydroxy-11$\beta$ méthyl-16$\alpha$ dioxo-3,20 prégnadiène-1,4 oïque-21.

3. Méthylamide de l'acide fluoro-6$\alpha$ hydroxy-11$\beta$ méthyl-16$\alpha$ dioxo-3,20 prégnadiène-1,4 oïque-21.

4. Ethylamide de l'acide fluoro-6$\alpha$ hydroxy-11$\beta$ méthyl-16$\alpha$ dioxo-3,20 prégnadiène-1,4 oïque-21.

5. Butylamide de l'acide fluoro-6$\alpha$ hydroxy-11$\beta$ méthyl-16$\alpha$ dioxo-3,20 prégnadiène-1,4 oïque-21.

6. Pentylamide de l'acide fluoro-6$\alpha$ hydroxy-11$\beta$ méthyl-16$\alpha$ dioxo-3,20 prégnadiène-1,4 oïque-21.

7. Hexylamide de l'acide fluoro-6$\alpha$ hydroxy-11$\beta$ méthyl-16$\alpha$ dioxo-3,20 prégnadiène-1,4 oïque-21.

8. Cyclohexylamide de l'acide fluoro-6$\alpha$ hydroxy-11$\beta$ méthyl-16$\alpha$ dioxo-3,20 prégnadiène-1,4 oïque-21.

9. Pipéridide de l'acide fluoro-6$\alpha$ hydroxy-11$\beta$ méthyl-16$\alpha$ prégnadiène-1,4 oïque-21.

10. Morpholide de l'acide fluoro-6$\alpha$ hydroxy-11$\beta$ méthyl-16$\alpha$ dioxo-3,20 prégnadiène-1,4 oïque-21.

11. Diéthylamide de l'acide fluoro-6$\alpha$ hydroxy-11$\beta$ méthyl-16$\alpha$ dioxo-3,20 prégnadiène-1,4 oïque-21.

12. Benzylamide de l'acide fluoro-6$\alpha$ hydroxy-11$\beta$ méthyl-16$\alpha$ dioxo-3,20 prégnadiène-1,4 oïque-21.

13. (Méthyl-1 propyl)-amide de l'acide fluoro-6$\alpha$ hydroxy-11$\beta$ méthyl-16$\alpha$ dioxo-3,20 prégnadiène-1,4 oïque-21.

14. Diméthylamide de l'acide fluoro-6$\alpha$ hydroxy-11$\beta$ méthyl-16$\alpha$ dioxo-3,20 prégnadiène-1,4 oïque-21.

15. Ethylamide de l'acide fluoro-6$\alpha$ hydroxy-11$\beta$ méthyl-16$\alpha$ dioxo-3,20 prégnène-4 oïque-21.

16. Butylamide de l'acide fluoro-6$\alpha$ méthyl-16$\alpha$ trioxo-3,11,20 prégnadiène-1,4 oïque-21.

17. Diméthylamide de l'acide fluoro-6$\alpha$ méthyl-16$\alpha$ trioxo-3,11,20 prégnadiène-1,4 oïque-21.

18. Amide de l'acide difluoro-6$\alpha$,9$\alpha$ hydroxy-11$\beta$ méthyl-16$\alpha$ dioxo-3,20 prégnadiène-1,4 oïque-21.

19. Méthylamide de l'acide difluoro-6α,9α hydroxy-11β méthyl-16α dioxo-3,20 prégnadiène-1,4 oïque-21.

20. Diméthylamide de l'acide difluoro-6α,9α hydroxy-11β méthyl-16α dioxo-3,20 prégnadiène-1,4 oïque-21.

21. Diéthylamide de l'acide difluoro-6α,9α hydroxy-11β méthyl-16α dioxo-3,20 prégnadiène-1,4 oïque-21.

22. Amide de l'acide chloro-9α fluoro-6α hydroxy-11β méthyl-16α dioxo-3,20 prégnadiène-1,4 oïque-21.

23. Méthylamide de l'acide chloro-9α fluoro-6α hydroxy-11β méthyl-16α dioxo-3,20 prégnadiène-1,4 oïque-21.

24. Morpholide de l'acide chloro-9α fluoro-6α hydroxy-11β méthyl-16α dioxo-3,20 prégnadiène-1,4 oïque-21.

25. Diméthylamide de l'acide chloro-9α fluoro-6α hydroxy-11β méthyl-16α dioxo-3,20 prégnadiène-1,4 oïque-21.

26. Diéthylamide de l'acide chloro-9α fluoro-6α hydroxy-11β méthyl-16α dioxo-3,20-prégnadiène-1,4 oïque-21.

27. Pentylamide de l'acide chloro-2 fluoro-6α hydroxy-11β méthyl-16α dioxo-3,20 prégnadiène-1,4 oïque-21.

28. Cyclohexylamide de l'acide chloro-9α difluoro-6α,11β méthyl-16α dioxo-3,20 prégnadiène-1,4 oïque-21.

29. Butylamide de l'acide dichloro-9α,11β fluoro-6α méthyl-16α dioxo-3,20 prégnadiène-1,4 oïque-21.

30. Amide de l'acide fluoro-9α hydroxy-11β méthyl-16α dioxo-3,20 prégnadiène-1,4 oïque-21.

31. Hexylamide de l'acide fluoro-9α hydroxy-11β méthyl-16α dioxo-3,20 prégnadiène-1,4 oïque-21.

32. Butylamide de l'acide hydroxy-11β dioxo-3,20 prégnadiène-1,4 oïque-21.

33. Morpholide de l'acide hydroxy-11β dioxo-3,20 prégnadiène-1,4 oïque-21.

34. Méthylamide de l'acide hydroxy-11β dioxo-3,20 prégnadiène-1,4 oïque-21.

35. Amide de l'acide hydroxy-11β dioxo-3,20 prégnadiène-1,4 oïque-21.

36. Compositions pharmaceutiques contenant, comme substance active, un ou plusieurs corticoïdes selon l'une quelconque des revendications 1 à 35.

37. Procédé de préparation de composés répondant à la formule générale I

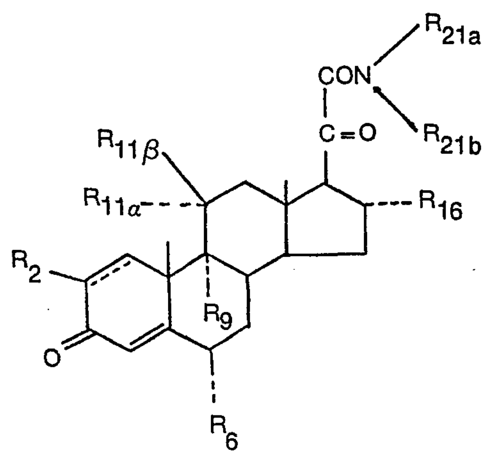

(I)

dans laquelle

la liaison <u>. . .</u> est une liaison simple ou une liaison double,

$R_2$ représente un atome d'hydrogène ou de chlore,

$R_6$ représente un atome d'hydrogène ou de fluor,

$R_{16}$ représente un atome d'hydrogène ou un radical méthyle,

$R_9$ représente un atome d'hydrogène, de fluor ou de chlore,

$R_{11\alpha}$ et $R_{11\beta}$ forment ensemble un groupe oxo, ou $R_{11\alpha}$ représente un atome d'hydrogène et $R_{11\beta}$ un groupe hydroxy, ou, lorsque $R_9$ est un atome de chlore, $R_{11\beta}$ est également un atome de fluor ou de chlore, et

$R_{21a}$ et $R_{21b}$ représentent chacun, indépendamment l'un de l'autre, un atome d'hydrogène, un radical alkyle contenant au plus 8 atomes de carbone ou un radical cyclo-alkyle, ou forment ensemble un radical alkylène contenant au plus 8 atomes de carbone et éventuellement interrompu par de l'oxygène ou de l'azote, procédé caractérisé en ce que, en opérant de manière connue:

a) on condense un acide carboxylique répondant à la formule générale II

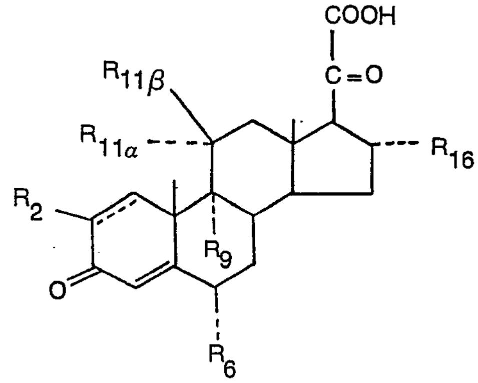

(II)

dans laquelle ...., $R_2$, $R_6$, $R_9$, $R_{11\alpha}$, $R_{11\beta}$ et $R_{16}$ ont les significations précédemment données, ou un dérivé réactif de cet acide, avec une amine répondant à la formule générale III

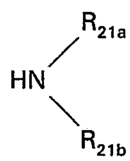

(III),

dans laquelle $R_{21a}$ et $R_{21b}$ ont les significations indiquées ci-dessus, ou

b) pour préparer des corticoïdes dans lesquels $R_9$ désigne un atome de chlore on fixe de l'acide hypochloreux, du chlorure de fluor ou du chlore sur la double liaison 9(11) d'un composé répondant à la formule générale IV

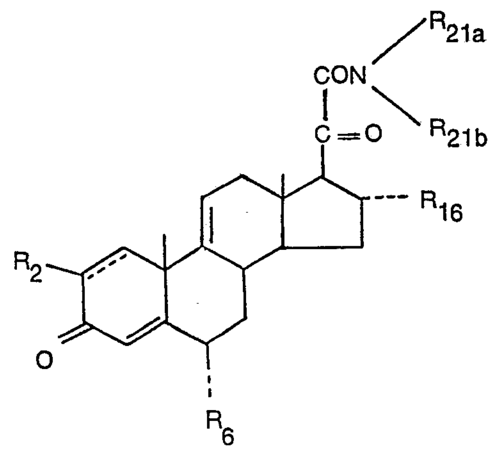

(IV),

dans laquelle $R_2$, $R_6$, $R_{16}$, $R_{21a}$ et $R_{21b}$ ont les significations précédemment données, ou

c) pour préparer des corticoïdes dans lesquels $R_{11\beta}$ désigne un groupe hydroxy et $R_9$ un atome de fluor ou un atome de chlore on ouvre le noyau époxydique en 9,11 d'un composé répondant à la formule générale V

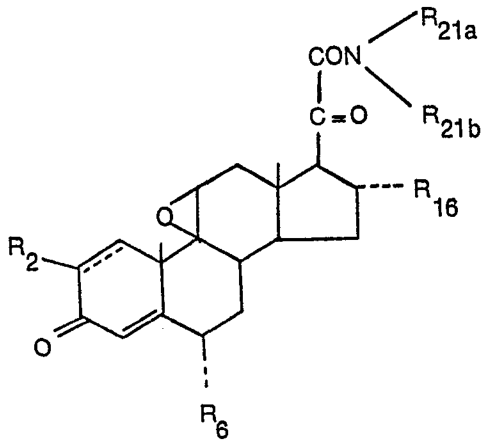

(V)

par du fluorure d'hydrogène ou du chlorure d'hydrogène.